# FASCICULE DE BREVET EUROPEEN

(11) **EP 0 589 772 B1**
(45) Date de publication et mention de la délivrance du brevet: **02.11.1995**
(21) Numéro de dépôt: 93402295.5
(22) Date de dépôt: 20.09.1993
(51) Int. Cl.: G01N 3/42, G01N 3/52, G01N 33/42

(54) **Appareillage d'essai de sol du type à plaque et véhicule équipé d'un tel appareillage**
Bodenprüfvorrichtung mit einer Druckplatte und Fahrzeug mit dieser Prüfvorrichtung
Plate apparatus for mechanical testing of soils and vehicle equipped with this apparatus

(30) Priorité: 21.09.1992 FR 9211221
(43) Date de publication de la demande: 30.03.1994
(73) Titulaire: COLAS S.A., F-92653 Boulogne-Billancourt Cédex (FR); Centre Expérimental de Recherches et d'Etudes du Bâtiment et des Travaux Publics (CEBTP), F-75015 Paris (FR)
(72) Inventeur: Chappat, Michel, F-78310 Maurepas (FR); Reymonet, Jean-Pierre, F-78150 Le Chesnay (FR); Chipart, Jean-Pierre, F-92120 Palaiseau (FR); Heritier, Bernard, F-91400 Orsay (FR)
(74) Mandataire: Michelet, Alain

(56) Documents cités:
- FR-A- 1 001 230
- FR-A- 2 584 186
- US-A- 4 116 041
- US-A- 4 726 239
- DATABASE WPI Week 7839, 1978 Derwent Publications Ltd., London, GB; AN 78-H5938A & SU-A-579 370 (RULKEVICH) 15 Décembre 1977
- SOVIET INVENTIONS ILLUSTRATED Section EI, Week C40, 12 Novembre 1980 Derwent Publications Ltd., London, GB; Class SO3, AN J6615 & SU-A-715 968 (ROST. ENG. CONST. INST.) 15 Février 1980
- PATENT ABSTRACTS OF JAPAN vol. 15, no. 37 (P-1159)29 Janvier 1991 & JP-A-02 275 339 (TOSHIBA) 9 Novembre 1990
- DATABASE WPI Section EI, Week 9013, 1990 Derwent Publications Ltd., London, GB; Class SO3, AN 90091505 & CN-A-1 032 398 (MECHANICAL ROAD-CON.) 4 Décembre 1989

## Description

La présente invention est relative à un appareillage d'essai de sol du type à plaque, ainsi qu'a un véhicule équipé d'un tel appareillage.

Les appareillages du type à plaque pour la mesure de compacité de sols sont déjà connus. On distingue parmi eux les appareillages permettant de réaliser des essais statiques tels que décrits dans le document SOVIET INVENTIONS ILLUSTRATED Section EI, Week C40, 12 Novembre 1980 Derwent Publications Ltd, London GB; Class SO3, ANJ6615 & SU-A-715 968 et les appareillages permettant de réaliser des essais dynamiques tels que décrits dans le document US-A-4 116 041. Un appareillage classique pour un essai de sol statique est par exemple constitué d'une plaque dite de chargement et d'un appui massif, tel qu'un camion lesté. La plaque est disposée sous le châssis du camion. Un vérin est interposé entre la plaque et ledit chassis et vient exercer sur ladite plaque, en prenant appui sur le châssis du camion, la force souhaitée. La compacité du sol se déduit d'une mesure de l'enfoncement ainsi obtenu.

Pour réaliser des essais dynamiques, un matériel couramment utilisé est par exemple celui commercialisé par le laboratoire des Ponts et Chaussées sous la dénomination de Dynaplaque. Il comprend une potence montée sur une plaque destinée à être posée sur le sol. Une plaque d'essai portant une masse est lâchée du haut de la potence sur la plaque au sol. La plaque d'essai rebondit sur des ressorts solidaires, les uns, de ladite plaque d'essai, les autres de ladite plaque au sol. Sur cette plaque sont montés plusieurs ressorts qui portent eux mêmes une plaque d'essai. Une masse est lâchée du haut de la potence sur la plaque portée par les ressorts. La mesure de la compacité du sol se déduit d'une observation des rebonds des ressorts et de la masse lichée sur ceux-ci.

La présente invention propose quant à elle une structure qui permet à un opérateur de réaliser à partir d'un même matériel l'un ou l'autre de ces types d'essais.

On notera également que cette structure peut facilement équiper un véhicule léger dont la conduite ne nécessite pas de permis particulier. Elle offre donc aux opérateurs une grande autonomie dans leur travail. Ils peuvent réaliser seuls des essais statiques de sol, sans qu'il ne soit nécessaire de faire intervenir un poids lourd et un conducteur pour déplacer celui-ci sur le site.

La présente invention a donc pour objet un appareillage d'essai de sol comportant une plaque d'appui destinée à venir en appui sur le sol à tester, des moyens de sollicitation mécaniques de ladite plaque et des moyens pour la mesure de données relatives à ces sollicitations, desquelles on déduit des valeurs représentatives de la compacité du sol testé, caractérisé en ce qu'il comporte pour une même plaque d'appui des moyens de sollicitation de type dynamique et des moyens de sollicitation de type statique, ces moyens étant susceptibles de solliciter de façon indépendante ladite plaque d'appui.

Cet appareillage est avantageusement complété par les différentes caractéristiques suivantes prises seules ou selon toutes leurs combinaisons techniquement possibles:
- les moyens de sollicitation de type dynamique comprennent un ensemble mobile chargé et destiné à être lâché d'une certaine hauteur sur la plaque d'appui au sol, ainsi que des moyens ressorts interposés entre la charge dudit ensemble mobile et ladite plaque d'appui et en ce que les moyens de sollicitation de type statique comportent des moyens du type vérin pour exercer sur ladite plaque d'appui un effort statique axial;
- l'ensemble mobile comporte une plaque portant la charge dudit ensemble mobile;
- une plaque intermédiaire sur laquelle viennent porter les moyens ressorts lorsque l'ensemble mobile est lâché; un bras étant monté entre la plaque d'appui et cette plaque intermédiaire pour transmettre axialement par rapport à la plaque d'appui des efforts susceptibles d'être exercés sur lui vers chacune de ses extrémités par des moyens du type vérins;
- les moyens du type vérin peuvent être désolidarisés des extrémités dudit bras sur lesquelles ils sont susceptibles d'exercer des efforts, de façon que lors d'un essai dynamique, la plaque d'appui et ledit bras soient libres par rapport auxdits moyens de type vérin;
- les extrémités des moyens du type vérin par lesquelles des efforts sont susceptibles d'être exercés sur les extrémités dudit bras sont munies de lumières de guidage se terminant par une butée supérieure et une butée inférieure et dans lesquelles peuvent coulisser latéralement les extrémités dudit bras, entre une position où une telle extrémité est en appui sur ledit bras par sa butée supérieure et une autre position où ledit bras est porté par ladite butée inférieure pour la remontée de la plaque;
- la liaison entre le bras précité et la plaque d'appui comprend une liaison rotulaire entre ledit bras et un axe de transmission des efforts à ladite plaque;
- les moyens d'essais dynamiques, comprennent encore une potence en haut de laquelle l'ensemble mobile chargé est destiné à être hissé, ledit ensemble mobile étant muni de moyens permettant de le guider en coulissement par rapport aux bras latéraux de ladite potence;
- les bras latéraux de ladite potence sont des rails creux servant de rails de guidage pour des galets dont est muni latéralement l'ensemble mobile;
- les bras de ladite potence servent également de rail de guidage pour des galets d'un chariot de levage destiné à permettre de relever l'ensemble mobile une fois celui tombé jusqu'à la partie supérieure de la potence;
- le chariot de levage est muni de mâchoires destinées à se refermer sur des pions de levage complémentaires solidaires de l'ensemble mobile, pour lever ledit ensemble mobile;
- les mâchoires sont commandées par des moyens du type vérin;
- des moyens sont également prévus pour détecter la présence du chariot de levage au niveau de l'ensemble mobile;
- la charge de l'ensemble mobile est réglable;
- les corps du ou des vérins des moyens d'essais statiques sont solidarisés des bras de la potence des moyens d'essais dynamiques.

L'invention a encore pour objet un véhicule équipé d'un appareillage du type précité. Ce véhicule est avantageusement complété par les différentes caractéristiques suivantes prises seules ou selon toutes leurs combinaisons techniquement possibles:
- la potence de l'appareillage est solidaire de son châssis;
- ledit appareillage est disposé à l'arrière dudit véhicule, sa suspension arrière ayant été à cet effet renforcée;
- ledit véhicule est lesté en sa partie vers ledit appareillage;
- la charge totale dudit véhicule est de l'ordre de 2 tonnes;
- le véhicule peut être de type classique ou à quatre roues motrices.

La description qui suit d'un mode de réalisation particulier de l'invention est purement illustrative et non limitative. Elle doit être lue en regard des dessins annexés sur lesquels:
La Figure 1 est une vue de côté d'un véhicule équipé d'un appareillage d'essai de sol conforme à l'invention;
La Figure 2 est une vue en coupe de l'appareillage d'essai de sol équipant le véhicule de la Figure 1;
La Figure 3 est une vue en coupe selon la ligne III-III de la Figure 2;
La Figure 4, enfin, est une vue en coupe selon la ligne IV-IV de la Figure 2.

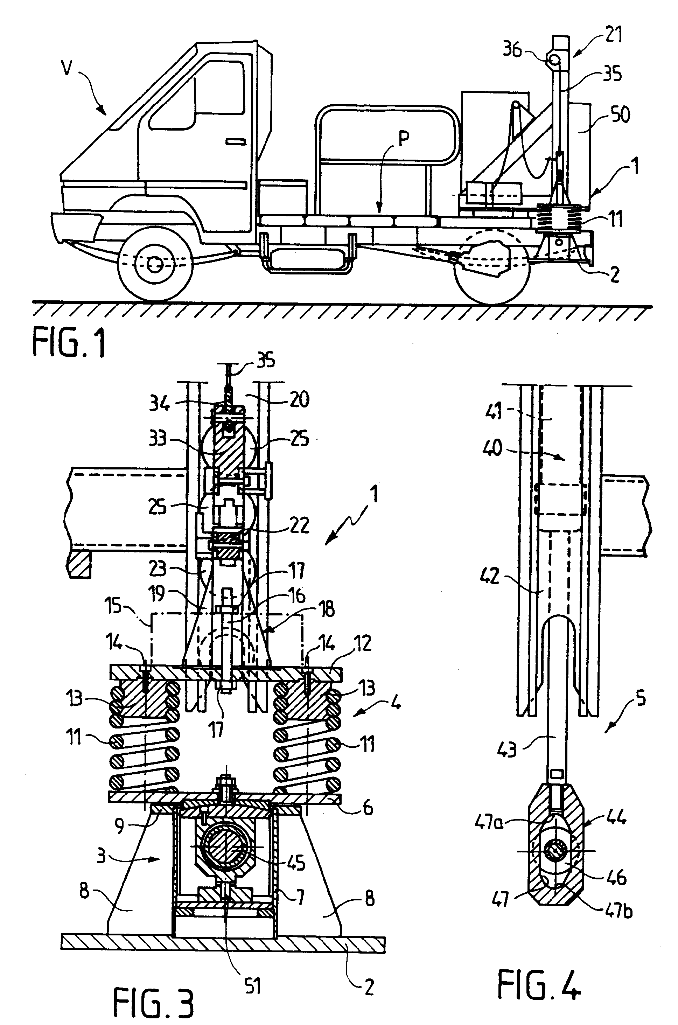

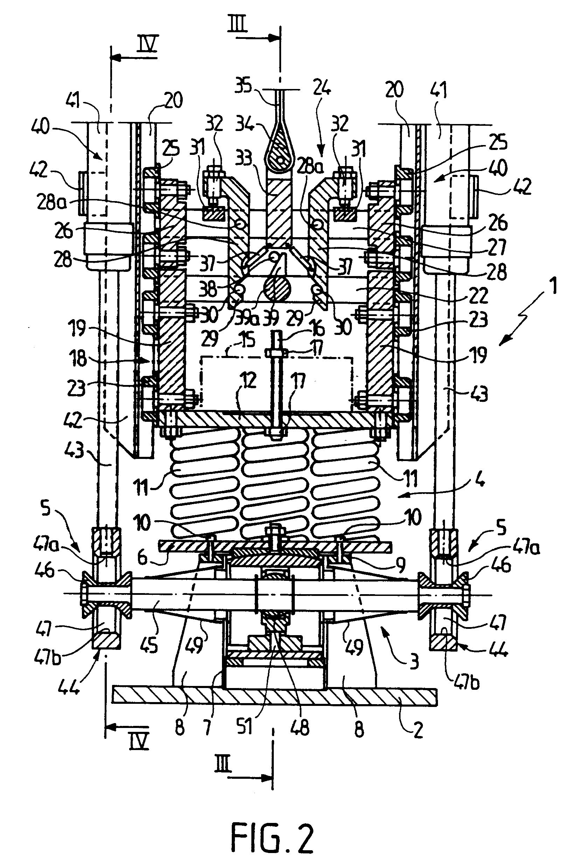

Le véhicule V à plate-forme P représenté sur la Figure 1 est un véhicule utilitaire du type léger (2 tonnes), dont la conduite ne nécessite pas de permis de conduire poids lourds. Il est équipé à l'arrière de sa plate-forme P d'un appareillage d'essai de sol 1 conforme à l'invention. Cet appareillage a été plus particulièrement représenté sur les Figures 2 à 4. Il comprend principalement une plaque 2 rigide de contour circulaire destinée à venir en appui sur le sol lors d'un essai. Cette plaque 2 est reliée par un ensemble de moyens de liaison 3, d'une part a un sous-appareillage 4 pour la réalisation des essais dynamiques, et d'autre part à un sous-appareillage 5 pour la réalisation des essais statiques.

Le sous-appareillage 4 comporte une plaque intermédiaire 6 à laquelle la plaque 2 est reliée au moyen d'un cylindre d'appui 7 et de nervures de rigidification 8 ayant une forme classique de trapèze rectangle prenant appui sur le cylindre 7, ainsi que par leur grande base sur le disque 2. La plaque 6 est un disque de plus petit diamètre que la plaque ou disque d'appui 2. Le disque 2 est d'une pièce avec lesdites nervures de rigidification 8 et un plateau 9 également de contour circulaire, mais de diamètre plus petit que le disque 2, et par lequel lesdites nervures 8 sont reliées entre elles au niveau de leurs petites bases ou bases supérieures. Ce plateau 9 porte le disque intermédiaire 6, auquel il est relié par des vis 10. La tête des vis 10 est en appui sur ledit disque intermédiaire 6, le corps desdites vis 10 traversant le disque 6, ainsi qu'au moins partiellement le plateau 9. Leurs filetages coopèrent avec des filetages complémentaires dont sont munis les alésages du plateau 9 les recevant.

Le sous appareillage 4 comporte encore un ensemble mobile 18 destiné à être lâché sur cette plaque 6. Cet ensemble 18 comprend un disque 12 faisant face à la plaque 6 et de même diamètre que celle-ci. Sur ce disque 12 sont montés six ressorts hélicoïdaux 11 répartis en cercle régulièrement sur ledit disque 12 et s'étendant dudit disque 12, vers la plaque 6 intermédiaire.

Ces ressorts 11 sont reliés au disque 12 au moyen de tenons cylindriques 13 solidarisés par des vis 14 du disque 12 et sur lesquels sont emboîtés à force lesdits ressorts 11. Une charge réglable 15 est montée sur ledit disque d'extrémité 12 auquel elle est solidarisée au moyen d'un goujon 16 traversant axialement le disque 12 et la charge 15, et de deux boulons 17 entre lesquels le disque 12 et la charge 15 sont maintenus.

De l'autre côté, le disque 12 est solidaire de deux pieds 19 ayant une forme trapézoïdale à leur base et coulissant chacun le long d'un des bras 20 d'une potence 21 (plus particulièrement représentée sur la Figure 1), du haut de laquelle l'ensemble mobile 18 est destinée à être lâchée. Les deux pieds 19 sont reliés entre eux à leur partie supérieure au moyen de deux poutres 22. Ils sont disposés de part et d'autre de la charge 15. Les bras 20 de la potence 21 sont des rails creux par rapport auxquels les pieds 19 sont guidés au moyen de galets roulants 23. Ces rails creux 20 servent également de rail de guidage pour les galets 25 d'un chariot de relevage 24. Celui-ci comporte principalement deux branches 26 reliées entre elles par deux poutres 27 formant entretoises médianes. Les branches 26 prolongent, lorsque le chariot de relevage 24 est descendu sur l'ensemble mobile 18, les pieds 19 de celle-ci.

Les poutres 27 du chariot de relevage portent deux mâchoires 28 s'étendant dans l'espace entre lesdites deux poutres 27, ainsi que l'espace entre lesdites poutres 22, lorsque le chariot 24 est en position descendue. Ces mâchoires 28 sont articulées en pivotement sur lesdites poutres 27 par deux axes 28a perpendiculaires au plan principal de la potence 21. Elles se terminent à leurs extrémités les plus proches du disque 12 par chacune un décrochement 29, destiné à crocheter un pion 30, deux pions 30 étant disposés en entretoise entre lesdites poutres 22, au droit des axes 28a. A leurs extrémités opposées à ces décrochements 29, ces deux mâchoires 28 ont des formes en L renversé, dont les branches supérieures s'étendent en saillie par rapport aux branches principales desdites mâchoires 28, chacune vers le bras 20 de la potence 21 qui est du même côté qu'elle par rapport au plan de symétrie de ladite potence 21. Ces branches d'extrémité portent chacune un poinçon 32 destiné à venir en butée sur des plots 31 entretoisant les poutres 27 du chariot 24. Ces plots 31 sont des plots de sécurité qui évitent, lorsque la fermeture des mâchoires 28 est commandée à vide, c'est-à-dire sans que la masse ne se trouve à leur niveau, qu'il y ait endommagement des mâchoires 28 et du chariot de relevage 24.

Les deux poutres 27 sont encore solidarisées d'un bras 33 s'étendant dans le plan de symétrie de la potence 21 perpendiculairement par rapport auxdites poutres 27 et de la partie médiane des mâchoires 28, vers le haut de ladite potence 21. Ce bras 33 est muni a son extrémité supérieure d'une poulie 34 sur laquelle est passé un câble fermé 35 enroulé à son autre extrémité sur un treuil 36 (voir Figure 1) monté en partie haute de la potence 21. A l'autre extrémité de ce bras 33 sont articulés deux vérins 37 eux-mêmes articulés à leurs autres extrémités chacun sur la partie inférieure d'une des mâchoires 28. Ce sont ces vérins 37 qui lorsqu'ils sont actionnés commandent l'ouverture et la fermeture desdites mâchoires 28. A cette même extrémité du bras 33 est également articulé un contacteur 38 à pivotement. Les poutres 22 de l'ensemble mobile 18 sont munies d'une butée 39 solidaire d'une entretoise 39a cylindrique montée entre lesdites poutres 22. Un pan de ladite butée 39 est incliné pour former une rampe pour ledit contacteur 38. Lorsque le chariot 24 est descendu sur l'ensemble mobile 18, le contacteur 38 vient glisser le long de la rampe formée par ce pan incliné de la butée 39 et pivoter ainsi d'une position de repos à une position commandant la fermeture des mâchoires 28.

Le sous-appareillage d'essai statique 5 comprend quant à lui principalement deux vérins 40 s'étendant le long chacun d'un des bras 20 de la potence 21. Les corps 41 desdits vérins 40 sont solidarisés desdits bras 20 par des moyens de liaison et un carénage ouvert référencés dans leur ensemble par 42. Les tiges 43 desdits vérins 40 s'étendent desdits corps 41 vers la partie basse de l'ensemble de l'appareillage 1, c'est-à-dire vers la plaque 2. Les extrémités libres desdites tiges 43 se terminent par chacune une oreille de guidage 44. Ces deux oreilles de guidage 44 sont reliées entre elles par une poutre 45 se terminant à ses extrémités par des galets 46 coulissant dans des lumières 47 desdites oreilles de guidage 44. Ces lumières 47 se terminent à leurs extrémités par une butée supérieure 47a et une butée inférieure 47b. La poutre 45 est reliée par sa partie centrale à la fois à la plaque 2 et à la plaque intermédiaire 6. Les moyens de liaison 3 comprennent à cet effet entre lesdits disques 2 et 6 à l'intérieur du cylindre 7 une liaison rotulaire entre un axe 51 de transmission des efforts à la plaque 2 et ladite poutre 45. Des flasques souples de protection 49 sont montés de part et d'autre du cylindre 7 sur la poutre 45 pour protéger notamment la liaison rotulaire 48 par exemple des poussières extérieures.

Ces deux sous-appareillages 4 et 5 sont bien entendu complétés par des moyens de mesure du type capteur et par des moyens d'enregistrement. Le plancher central du véhicule V pourra être équipé de moyens informatiques et notamment d'une console de commande, celle-ci pouvant également se trouver à l'extérieur du véhicule pour permettre un contrôle visuel des essais. Le châssis du véhicule V est lesté en sa partie arrière par une masse. La suspension arrière du véhicule V est renforcée, l'équilibrage du véhicule V ayant été calculé en fonction notamment des efforts qui pourront être exercés dans des essais statiques entre l'arrière du véhicule et la plaque 2 d'appui sur le sol. L'ensemble des vérins de l'appareillage est commandé hydrauliquement. Un grillage de protection 50 (voir Figure 1) évite les risques d'accident lors des chutes de la charge mobile 18.

Le fonctionnement d'un tel appareillage 1 va maintenant être décrit. Lorsque le véhicule se déplace, ou de façon plus générale lorsqu'il n'est pas utilisé pour faire des essais, les tiges 43 des vérins 40 sont rentrées dans les corps des vérins 41. La plaque intermédiaire 6, les ressorts 11, et la plaque 12 sont relevés dans la position représentée sur la Figure 1, où ils se trouvent sensiblement au niveau du châssis du véhicule 1. Une fois arrivé sur le site d'essais, l'opérateur descend les tiges 43 des vérins pour mettre en appui la plaque 2 sur le sol.

S'il désire réaliser un essai dynamique, il descend ces tiges 43 de façon que les galets 46 soient sensiblement disposés au centre des lumières 47 des oreilles 44, libres de coulisser dans ces dernières. Le chariot 24 étant, avec l'ensemble 18, en position relevée, en haut de la potence 21, l'opérateur commande l'ouverture des mâchoires 28. Les vérins 37 sont alors actionnés en extension, les mâchoires 28 pivotant autour de leurs axes 28a et lâchant l'ensemble mobile 18. La plaque 12 chargée et les ressorts 11 tombent sur la plaque 6, qui transmet à la plaque 2, par l'intermédiaire des moyens de liaison 3, des sollicitations sur le sol d'appui. La poutre 45 et ses galets 46 étant libres de coulisser par rapport aux extrémités 44 des vérins 40, le sous-appareillage statique 5 n'entrave aucunement le mouvement d'enfoncement de la plaque 2 par rapport au sol. Les mesures faites par les capteurs sur le rebond de l'ensemble mobile 18 et les rebonds des ressorts 11 permettent de façon classique de calculer des valeurs représentatives de la compacité du sol. Une fois l'essai réalisé et les mesures enregistrées, le treuil est actionné pour descendre le chariot 24 sur l'ensemble mobile 18. L'arrivée du chariot 24 au niveau de l'ensemble mobile 18 sera détectée par le contacteur 38 en butée sur le pan incliné de la butée 39. Les vérins 37 seront rétractés pour refermer les mâchoires 28 sur les pions 30. Une fois cette opération effectuée, le câble 35 sera enroulé autour de son treuil, de façon à faire remonter le chariot de levage 24, les pieds 19, la plaque 12 et les ressorts 11.

Lorsque maintenant un opérateur souhaite réaliser un essai statique, il descendra les tiges 43 des vérins 40 de façon que les extrémités supérieures 47a des oreilles 44 viennent en appui sur les galets 46. Le vérin 40 développera ainsi une force entre les moyens de liaison 3 et donc la plaque 2 en appui sur le sol et le châssis du véhicule V. De façon classique, les mesures de l'enfoncement de la plaque 2 permettront d'obtenir des valeurs caractéristiques de la compacité du sol testé.

## Revendications

1. Appareillage d'essai de sol comportant une plaque d'appui (2) destinée à venir en appui sur le sol à tester, des moyens de sollicitations mécaniques (4, 5) de ladite plaque (2) et des moyens pour la mesure de données relatives à ces sollicitations, desquelles l'on déduit des valeurs représentatives de la compacité du sol testé, caractérisé en ce qu'il comporte pour une même plaque d'appui (2) des moyens de sollicitation de type dynamique (4) et des moyens de sollicitation de type statique (5), ces moyens (4, 5) étant susceptibles de solliciter de façon indépendante ladite plaque d'appui (2).

2. Appareillage selon la revendication 1, caractérisé en ce que les moyens (4) de sollicitation de type dynamique comprennent un ensemble mobile (18) chargé destiné à être lâché d'une certaine hauteur sur la plaque d'appui au sol (2), ainsi que des moyens ressorts interposés entre la charge dudit ensemble mobile (18) et ladite plaque d'appui (2) et en ce que les moyens (5) de sollicitation de type statique comportent des moyens (40) du type vérin pour exercer sur ladite plaque d'appui (2) un effort statique axial.

3. Appareillage selon la revendication 2, caractérisé en ce que l'ensemble mobile (18) comporte une plaque portant la charge dudit ensemble mobile (18).

4. Appareillage selon l'une des revendications 2 ou 3, caractérisé en ce qu'il comporte une plaque intermédiaire (6) sur laquelle viennent porter les moyens ressorts lorsque l'ensemble mobile (18) est chargé, un bras (45) étant monté entre la plaque d'appui (2) et cette plaque intermédiaire (6) pour transmettre axialement par rapport à la plaque d'appui (2) des efforts susceptibles d'être exercés sur lui vers chacune de ses extrémités (46) par des moyens du type vérins (40).

5. Appareillage selon la revendication 4, caractérisé en ce que les moyens du type vérin (40) peuvent être désolidarisés des extrémités (46) dudit bras (45) sur lesquelles ils sont susceptibles d'exercer des efforts, de façon que lors d'un essai dynamique, la plaque d'appui (2) et ledit bras (45) soient libres par rapport auxdits moyens de type vérin (40).

6. Appareillage selon la revendication 5, caractérisé en ce que les extrémités des moyens du type vérin (40) par lesquelles des efforts sont susceptibles d'être exercés sur les extrémités (46) dudit bras (45) sont munies de lumières de guidage (47) se terminant par une butée supérieure (47a) et une butée inférieure (47b) et dans lesquelles peuvent coulisser latéralement les extrémités (46) dudit bras (45), entre une position où une telle extrémité desdits moyens du type vérin (40) est en appui sur ledit bras (45) par sa butée supérieure (47a) et une autre position où ledit bras (45) est porté par ladite butée inférieure (47b) pour la remontée de la plaque (2).

7. Appareillage selon la revendication 6, caractérisé en ce que la liaison entre le bras (45) précité et la plaque d'appui (2) comprend une liaison rotulaire (48) entre ledit bras (45) et un axe de transmission (51) des efforts à ladite plaque (2).

8. Appareillage selon la revendication 2 prise seule ou en combinaison avec l'une quelconque des revendications 3 à 7, caractérisé en ce que les moyens (4) d'essai dynamique, comprennent encore une potence (21) en haut de laquelle l'ensemble mobile (18) chargé est destiné à être hissé, ledit ensemble mobile (18) étant muni de moyens (23) permettant de le guider en coulissement par rapport aux bras latéraux (20) de ladite potence (21).

9. Appareillage selon la revendication 8, caractérisé en ce que les bras latéraux (20) de ladite potence (21) sont des rails creux servant de rails de guidage pour des galets (23) dont est muni latéralement l'ensemble mobile (18).

10. Appareillage selon la revendication 9, caractérisé en ce que les bras (20) de ladite potence (21) servent également de rail de guidage pour des galets (25) d'un chariot de levage (24) destiné à permettre de relever l'ensemble mobile (18) une fois celui-ci tombé, jusqu'à la partie supérieure de la potence (21).

11. Appareillage selon la revendication 10, caractérisé en ce que ce chariot de levage (24) est muni de mâchoires (28) destinées à se refermer sur des pions (30) de levage complémentaires solidaires de l'ensemble mobile (18), pour lever ledit ensemble mobile (18).

12. Appareillage selon la revendication 11, caractérisé en ce que ces mâchoires (28) sont commandées par des moyens (37) du type vérin.

13. Appareillage selon l'une quelconque des revendications 7 et suivantes, caractérisé en ce que des moyens (38, 39) sont également prévus pour détecter la présence du chariot de levage (24) au niveau de l'ensemble mobile (18).

14. Appareillage selon la revendication 2 prise seule ou en combinaison avec l'une quelconque des revendications 3 et suivantes, caractérisé en ce que la charge de l'ensemble mobile (18) est réglable.

15. Appareillage selon la revendication 8 prise seule ou en combinaison avec l'une quelconque des revendications 8 et suivantes, caractérisé en ce que les corps (41) du ou des vérins des moyens d'essai statique (5) sont solidarisés des bras (20) de la potence (21) des moyens d'essai dynamique (4).

16. Véhicule caractérisé en ce qu'il comporte un appareillage (1) selon l'une quelconque des revendications précédentes.

17. Véhicule selon la revendication 16, caractérisé en ce que la potence (21) de l'appareillage est solidaire de son châssis.

18. Véhicule selon l'une quelconque des revendications 16 ou 17, caractérisé en ce que ledit appareillage (1) est disposé à l'arrière de celui-ci, sa suspension arrière ayant été à cet effet renforcée.

19. Véhicule selon l'une quelconque des revendications 16 à 18, caractérisé en ce qu'il est lesté en sa partie vers ledit appareillage (1).

20. Véhicule selon l'une quelconque des revendications 16 et suivantes, caractérisé en ce que la charge totale dudit véhicule est de l'ordre de 2 tonnes.

## Patentansprüche

1. Bodenprüfvorrichtung, mit einer Druckplatte (2), die dazu bestimmt ist, zur Auflage auf den zu testenden Boden zu kommen, mit mechanischen Belastungsmitteln (4,5) für die besagte Platte (2) und mit Meßmitteln für Daten bezüglich dieser Belastungen, von denen man die Festigkeit des getesteten Bodens repräsentierende Werte ableitet, dadurch gekennzeichnet, daß sie für eine selbe Druckplatte (2) Belastungsmittel dynamischen Typs (4) und Belastungsmittel statischen Typs (5) aufweist, die (4,5) geeignet sind, in unabhängiger Weise die besagte Druckplatte zu belasten.

2. Vorrichtung nach Anspruch 1, dadurch gekennzeichnet, daß die Belastungsmittel dynamischen Typs eine belastete Bewegungseinheit (18), die dazu ausgebildet ist, von einer bestimmten Höhe auf die Bodendruckplatte (2) gelassen zu werden, sowie zwischen der Last der besagten Bewegungseinheit (18) und der besagten Druckplatte (2) Federmittel aufweisen, und daß die Belastungsmittel statischen Typs zylinderartige Mittel (40) zur Ausübung eines statischen axialen Drucks auf die besagte Druckplatte (2) aufweisen.

3. Vorrichtung nach Anspruch 2, dadurch gekennzeichnet, daß die Bewegungseinheit (18) eine Platte aufweist, welche die Last der besagten Bewegungseinheit (18) trägt.

4. Vorrichtung nach einem der Ansprüche 2 oder 3, dadurch gekennzeichnet, daß sie eine Zwischenplatte (6) aufweist, auf der die Federmittel zum Tragen kommen, wenn die Bewegungseinheit (18) belastet wird, wobei ein Arm (45) zwischen der Druckplatte (2) und dieser Zwischenplatte (6) angeordnet ist, so daß bezüglich der Druckplatte (2) axial Drücke übertragen werden, die auf ihn gegen jeden seiner Enden (46) mittels der zylinderartigen Mittel (40) ausgeübt werden können.

5. Vorrichtung nach Anspruch 4, dadurch gekennzeichnet, daß die zylinderartigen Mittel (40) von den Enden (46) des besagten Armes (45) getrennt werden können, auf die sie Drücke ausüben können, derart, daß bei einer dynamischen Prüfung die Druckplatte (2) und der besagte Arm (45) gegenüber den besagten zylinderartigen Mitteln (40) frei werden.

6. Vorrichtung nach Anspruch 5, dadurch gekennzeichnet, daß die Enden der zylinderartigen Mittel (40), wodurch die Drücke auf die Enden (46) des besagten Armes (45) ausgeübt werden können, mit Führungslanglöchern (47) versehen sind, die in einem oberen Anschlag (47a) und einem unteren Anschlag (47b) enden, und in denen die Enden (46) des besagten Armes (45) seitlich gleiten können, zwischen einer Position, wo ein solches Ende der besagten zylinderartigen Mittel (40) mit seinem oberen Anschlag (47a) auf den besagten Arm (45) aufliegt, und einer anderen Position, wo der besagte Arm (45) von dem besagten unteren Anschlag (47b) getragen ist, um die Platte (2) anzuheben.

7. Vorrichtung nach Anspruch 6, dadurch gekennzeichnet, daß die Verbindung zwischen dem vorgenannten Arm (45) und der Druckplatte (2) eine Kugelgelenkverbindung zwischen dem besagten Arm (45) und eine Transmissionsachse (51) für Drücke auf die besagte Platte (2) umfaßt.

8. Vorrichtung nach Anspruch 2 für sich allein oder in Kombination mit einem der Ansprüche 3 - 7, dadurch gekennzeichnet, daß die dynamischen Prüfmittel (4) noch eine Tragsäule (21) umfassen, auf deren oberen Teil die belastete Bewegungseinheit (18) hochgewunden werden kann, wobei die besagte Bewegungseinheit (18) mit Mitteln (23) versehen ist, welche deren Führung durch Gleiten relativ zu den Seitenarmen (20) der besagten Tragsäule (21) erlauben.

9. Vorrichtung nach Anspruch 8, dadurch gekennzeichnet, daß die Seitenarme (20) der besagten Tragsäule (21) als Hohlschienen ausgeführt sind, die als Führungsschienen für Führungsrollen (23) dienen, mit denen die Bewegungseinheit (18) seitlich ausgerüstet ist.

10. Vorrichtung nach Anspruch 9, dadurch gekennzeichnet, daß die Arme (20) der besagten Tragsäule (21) gleichfalls als Führungsschienen für Führungsrollen (25) eines Hebeschlittens (24) dienen, der zum Hochheben der einmal herabbewegten Bewegungseinheit (18) bis zum oberen Teil der Tragsäule (21) ausgebildet ist.

11. Vorrichtung nach Anspruch 10, dadurch gekennzeichnet, daß der Hebeschlitten (24) mit Backen (28) versehen ist, die zum Eingriff mit komplementären Hebeelementen (30) ausgebildet sind, die mit der Bewegungseinheit (18) baulich integriert sind, um die besagte Bewegungseinheit (18) zu heben.

12. Vorrichtung nach Anspruch 11, dadurch gekennzeichnet, daß die Backen (28) durch zylinderartige Mittel (37) betätigt werden.

13. Vorrichtung nach einem der Ansprüche 7 und folgende, dadurch gekennzeichnet, daß ebenfalls Mittel (38,39) zur Feststellung der Anwesenheit des Hebeschlittens (24) auf gleicher Höhe wie die Bewegungseinheit (18) vorgesehen sind.

14. Vorrichtung nach Anspruch 2 für sich allein genommen oder in Kombination mit einem der Ansprüche 3 und folgende, dadurch gekennzeichnet, daß die Last der Bewegungseinheit (18) einstellbar ist.

15. Vorrichtung nach Anspruch 8 für sich allein genommen, oder in Kombination mit einem der Ansprüche 8 und folgende, dadurch gekennzeichnet, daß die Rümpfe (41) des oder der Zylinder der statischen Prüfmittel (5) mit den Armen (20) der Tragsäule (21) der dynamischen Prüfmittel (4) baulich integriert sind.

16. Fahrzeug, dadurch gekennzeichnet, daß es eine Vorrichtung (1) gemäß einem der vorangehenden Ansprüche aufweist.

17. Fahrzeug nach Anspruch 16, dadurch gekennzeichnet, daß die Tragsäule (21) der Vorrichtung mit seinem Chassis baulich integriert ist.

18. Vorrichtung nach einem der Ansprüche 16 oder 17, dadurch gekennzeichnet, daß die besagte Vorrichtung (1) auf dessen rückwärtigem Teil angeordnet ist, wobei die rückwärtige Aufhängung zu diesem Zweck verstärkt ist.

19. Fahrzeug nach einem der Ansprüche 16 - 18, dadurch gekennzeichnet, daß es im Bereich der besagten Vorrichtung (1) mit Ballast versehen ist.

20. Fahrzeug nach einem der Ansprüche 16 und folgende, dadurch gekennzeichnet, daß die Gesamtlast des besagten Fahrzeugs in der Größenordnung von 2 Tonnen liegt.

## Claims

1. An apparatus for the testing of soils, comprising a bearing plate (2) designed for bearing on the soil to be tested, mechanical stressing means (4, 5) of the said plate (2) and means for the measurement of data related to these stresses, which enable to derive values representative of the compactness of the soil thus tested, characterised in that it contains for a given bearing plate (2) dynamic stressing means (4) as well as static stressing means (5), the said means (4, 5) being liable to impose independent stresses onto the said bearing plate (2).

2. An apparatus according to the claim 1, characterised in that the dynamic stressing means (4) comprise a loaded mobile assembly (18), designed to be dropped from a given height onto the bearing plate on the soil (2), as well as spring-loaded means interposed between the load of the said mobile assembly (18) and the said bearing plate (2) and in that the static stressing means (5) comprise jack-type means (40) in order to exert an axial static stress on the said bearing plate (2).

3. An apparatus according to the claim 2, characterised in that the mobile assembly (18) comprises a plate carrying the load of the said mobile assembly (18).

4. An apparatus according to one of the claims 2 or 3, characterised in that it contains an intermediate plate (6) subject to the weight of the spring-loaded means whenever the mobile assembly (18) is mounted, whereby an arm (45) is located between the bearing plate (2) and this intermediate plate (6) in order to transmit, axially with respect to the bearing plate (2), stresses liable to be exerted onto the said arm towards each of its ends (46) by jack-type means (40).

5. An apparatus according to the claim 4, characterised in that the jack-type means (40) can be separated from the ends (46) of the said arm (45) on which they are liable to exert stresses, so that during a dynamic test, the bearing plate (2) and the said arm (45) can be free with respect to the said jack-type means (40).

6. An apparatus according to the claim 5, characterised in that the ends of the jack-type means (40) through which stresses are liable to be exerted onto the ends (46) of the said arms (45) are fitted with guiding slots (47) terminated by an upper stop (47a) and a lower stop (47b) and in which the ends (46) of the said arm (45) may slide laterally, between a position where such an end of the said jack-type means (40) is bearing onto the said arm (45) with its upper stop (47a) and another position where the said arm (45) is carried by the said lower stop (47b) for the rising of the plate (2).

7. An apparatus according to the claim 6, characterised in that the link between the arm (45) mentioned above and the bearing plate (2) comprises an articulated link (48) between the said arm (45) and an axle (51) for the transmission of the stresses to the said plate (2).

8. An apparatus according to the claim 2, taken into consideration on its own or in conjunction with any of the claims 3 to 7, characterised in that the dynamic testing means (4) also contain a beam (21) at the top of which the mobile assembly (18), mounted, is designed for being winched, whereby the said mobile assembly (18) is fitted with means (23) to guide it while sliding with respect to the side arms (20) of the said beam (21).

9. An apparatus according to the claim 8, characterised in that the side arms (20) of the said beam (21) are hollow rails used as guiding rails for rollers (23) fitted laterally on the mobile assembly (18).

10. An apparatus according to the claim 9, characterised in that the arms (20) of the said beam (21) are also used as guiding rails for the rollers (25) of a lifting slide (24) designed for raising the mobile assembly (18) once dropped, up to the upper section of the beam (21).

11. An apparatus according to the claim 10, characterised in that the lifting truck (24) is fitted with jaws (28) designed for closing on mating lifting studs (30), connected rigidly to the mobile assembly (18) in order to raise the said mobile assembly (18).

12. An apparatus according to the claim 11, characterised in that these jaws (28) are actuated by jack-type means (37).

13. An apparatus according to any of the claims 7 and after, characterised in that means (38, 39) are also foreseen in order to detect the presence of the lifting truck (24) at the level of the mobile assembly (18).

14. An apparatus according to the claim 2, taken into consideration on its own or in conjunction with any of the claims 3 and following, characterised in that the load of the mobile assembly (18) is adjustable.

15. An apparatus according to the claim 8, taken into consideration on its own or in conjunction with any of the claims 8 and following, characterised in that the bodies (41) of the jack(s) of the static testing means (5) are connected rigidly to the arms (20) of the beam (21) of the dynamic testing means (4).

16. A vehicle characterised in that it contains an apparatus (1) according to any of the previous claims.

17. A vehicle according to the claim 6, characterised in that the beam (21) of the apparatus is connected rigidly with its chassis.

18. A vehicle according to any of the claims 16 or 17, characterised in that the said apparatus (1) is arranged at the rear of the former, whereby its rear suspension has been reinforced to this end.

19. A vehicle according to any of the claims 17 to 18, characterised in that it is ballasted at its section directed towards the said apparatus (1).

20. A vehicle according to any of the claims 16 and following, characterised in that the total load of the said vehicle is in the order of 2 tons.
